# EUROPÄISCHE PATENTSCHRIFT

(11) **EP 1 772 734 B1**
(45) Veröffentlichungstag und Bekanntmachung des Hinweises auf die Patenterteilung: **03.06.2009**
(21) Anmeldenummer: 06020937.6
(22) Anmeldetag: 05.10.2006
(51) Int. Cl.: G01N 33/68, G01N 33/50

(54) **Verfahren zur Bestimmung der Menge von zellulär ausgeschiedenen detektierbaren Stoffen**
Method for determining the amount of detectable substances released by cells
Procédé destiné à la détermination de la quantité de substances cellulaires éliminées pouvant être détectées

(30) Priorität: 05.10.2005 DE 102005048577
(43) Veröffentlichungstag der Anmeldung: 11.04.2007
(73) Patentinhaber: Autoimmun Diagnostika GmbH, 72479 Strassberg (DE)
(72) Erfinder: Schöllhorn, Volkmar, 72458 Albstadt (DE)
(74) Vertreter: Patentanwälte Ruff, Wilhelm, Beier, Dauster & Partner

(56) Entgegenhaltungen:
- EP-A2- 1 528 395
- PRESBER FRANZISKA: "Bestimmung spenderreaktiver, IFNgamma-produzierender Zellen vor und nach Nierentransplantation im ELISpot-Assay ? Zusammenhang mit frühen akuten Rejektionen und mit dem klinischen Ausgang"[Online] 10. August 2005 (2005-08-10), Seiten 1-118, XP002418018 Gefunden im Internet: URL:http://edoc.hu-berlin.de/dissertatione n/presber-franziska-2005-07-11/PDF/Presber .pdf> [gefunden am 2007-02-01]

## Beschreibung

Die Erfindung betrifft ein Verfahren zur Bestimmung der Menge von zellulär ausgeschiedenen detektierbaren Stoffen, insbesondere zur Diagnose und/oder Verfolgung der Therapie von Erkrankungen.

Das ELISA(Enzyme-Linked Immunosorbant Assay) ist ein immunologisches Nachweisverfahren für verschiedenste Biomoleküle, insbesondere für Proteine und Hormone. Das klassische ELISA-Verfahren wird als sogenanntes Sandwich-Verfahren mit zwei Antikörpern, die beide hochspezifisch für das nachzuweisende Antigen sind, durchgeführt, wobei ein Antikörper (Coating-Antikörper) auf einen festen Träger immobilisiert wird und ein zweiter Antikörper (Detection-Antikörper), insbesondere in Form eines Enzymkonjugats, das einen kolorimetrischen Nachweis erlaubt, nach einer gewissen Inkubationsphase hinzugegeben wird. Mit Hilfe des ELISA-Verfahrens können somit zellulär ausgeschiedene Stoffe nachgewiesen werden, wobei eine quantitative und auch qualitative Analyse der zellulären Aktivität -Intensität der zellulären Reaktion auf einen bestimmten exogenen Einfluß, beispielsweise auf ein Antigen- in vielen Fällen schwierig sein dürfte.

Eine Weiterentwicklung im Hinblick auf eine verbesserte Beurteilung der zellulären Aktivität stellt das sogenannte ELISA-Spot-Verfahren (ELlspot-Verfahren) dar, wobei auf einem Träger Abfangmoleküle immobilisiert werden, die zellulär ausgeschiedene Stoffe binden können. Durch die gleichzeitige Anwesenheit von die Zellen stimulierenden Fremdstoffen, insbesondere Antigenen, werden die zu untersuchenden Zellen zur Sezernation bzw. Abscheidung von spezifischen Stoffen, insbesondere Abwehr- und/oder Botenstoffe, veranlaßt, die von den immobilisierten Abfangmolekülen gebunden werden und zu einer die Aktivität einer einzelnen Zelle wiederspiegelnden Farbreaktion gebracht werden können. Diese Verfahren zur Bestimmung der zellulären Aktivität beruht auf der Messung der Anzahl von Zellen, allerdings ungeachtet der Tatsache, daß einzelne Zellen auf den gleichen Fremdstoff sehr unterschiedlich reagieren können, was sich insbesondere in der Menge der zellulär abgeschiedenen Stoffe manifestiert. Hieraus können sich durchaus Qualitätsabstufungen bezüglich der Aktivität einzelner Zellen ergeben, die mit dem konventionellen ELlspot-Verfahren nicht erfaßt werden.

Daher wurden in den vergangenen Jahren sehr viele Bemühungen unternommen, immunologische Verfahren zu entwickeln, die auch eine qualitative Aussagekraft im Hinblick auf zelluläre Aktivitäten ermöglichen. Ein weiterentwickeltes Verfahren ist insbesondere der DE 198 21 289 A1 zu entnehmen, bei welchem die Qualität der Aktivität einzelner Zellen durch Bestimmung der Größe der erzeugten Farbflächen (Spots) auf einer Untersuchungsfläche berücksichtigt wird. Das Verfahren beruht auf der Erkenntnis, daß aktivere Zellen größere Spots erzeugen als weniger aktive Zellen und daß die Größe der Spots proportional zur Aktivität der einzelnen Zellen ist.

In der DE 103 52 678 A1 wird ein weiteres leistungsstarkes Verfahren beschrieben, bei welchem die Messung der Qualität der Aktivität einzelner Zellen anhand der Größe und der Intensität der auf einer Untersuchungsfläche aufgetretenen Spots bestimmt wird. Somit wird dem Umstand Rechnung getragen, daß beispielsweise ein kleiner Spot, der eine intensive Färbung aufweist, eine stärkere zelluläre Reaktion widerspiegelt als ein großer Spot, der weniger intensiv gefärbt ist. Gewisse Schwierigkeiten bei der Evaluierung der zellulären Aktivität können sich jedoch in solchen Fällen ergeben, in denen eine Qualitätsbeurteilung und gegebenenfalls -differenzierung von Spots erforderlich ist, die Farbintensitäten aufweisen, die über dem technisch meßbaren Maximalwert der Farbintensität liegen. In diesen Fällen entspricht die gemessene Aktivität der Zellen nicht ihrer wirklichen Aktivität, wodurch sich ungenaue Meßergebnisse ergeben können. Weitere Probleme können sich insbesondere bei Vorliegen von Zellverbänden (Clustern) ergeben, die zu überlappenden Färbungen führen können, so daß in solchen Fällen eine Messung der Qualität der Aktivität einzelner Zellen zusätzlich erschwert wird.

Die Erfindung stellt sich somit die Aufgabe, ein Verfahren zur Bestimmung der Gesamtaktivität von Zellen bereitzustellen, das auch bei Auftreten intensiv gefärbter und insbesondere überlappender Farbflächen auf einer Untersuchungsfläche eine exakte und zuverlässige Beurteilung der zellulären Gesamtaktivität erlaubt, insbesondere hinsichtlich der Qualität der Gesamtaktivität der Zellen auf der Untersuchungsfläche.

Gegenstand der Erfindung ist ein Verfahren zur Bestimmung der Menge von zellulär ausgeschiedenen detektierbaren Stoffen, insbesondere zur Diagnose und/oder Verfolgung der Therapie von Erkrankungen, bei welchem auf mindestens einer Untersuchungsfläche eines Trägers von Zellen ausgeschiedene Stoffe mittels Abfangmolekülen anhand von gefärbten Flächen sichtbar gemacht werden, indem die Flächen in Einzelpunkte zerlegt und diese verarbeitet werden, wobei die Intensität der gesamten Färbung auf der Untersuchungsfläche quantitativ gemessen wird.

Unter dem Begriff "Gesamtaktivität" im Sinne der vorliegenden Erfindung soll die Gesamtintensität der Reaktion aller Zellen auf der gesamten Untersuchungsfläche auf einen definierten exogenen Einfluß, beispielsweise ein Antigen, verstanden werden.

Unter dem Begriff "Therapie" im Sinne der vorliegenden Erfindung sollen sowohl die Behandlung von Erkrankungen, die Behandlung von mit den Erkrankungen einhergehenden Befindlichkeitsstörungen sowie entsprechende Präventionsmaßnahmen verstanden werden.

Erfindungsgemäß wird die Untersuchungsfläche des Trägers in eine Vielzahl von Einzelpunkten zerlegt, die Farbintensität jedes Einzelpunktes getrennt gemessen und werden die gemessenen Intensitätswerte addiert. Es ist insbesondere vorgesehen, daß 1 bis 2 Millionen, insbesondere ca. 1,5 Millionen, Bildpunkte (Pixel) pro Untersuchungsfläche, beispielsweise durch eine Kamera, erfaßt werden. Für die Messung der Farbintensität eines Einzelpunktes auf der Untersuchungsfläche stehen zwischen 200 und 300, insbesondere zwischen 220 und 260, vorzugsweise ca. 256 Abstufungen (Grauwerte)zur Verfügung. Die Verarbeitung und Auswertung der Bildpunkte kann insbesondere mit einem Lesegerät vorgenommen werden. Vorzugsweise wird die Gesamtzahl der Bildpunkte sowie ihre Intensität mit Hilfe eines Image Analyzers gemessen. Somit ist eine Messung der gesamten Färbung, bezogen auf die Untersuchungsfläche oder einen bestimmten Teil davon, möglich. Als Referenzwert kann eine ungefärbte Stelle auf der Untersuchungsfläche, gegebenenfalls auch einer anderen Untersuchungsfläche, dienen. Das Maß für die Gesamtaktivität ergibt sich dann aus der Anzahl der angeregten (gefärbten) Pixel multipliziert mit dem Farbwert (beispielsweise Graustufe zwischen 0 und 256) jedes angeregten Pixels. Das Produkt wird zweckmäßigerweise durch 1000 geteilt, um einfach handhabbare Zahlenwerte (Einheiten) zu erhalten. Die Erfassung und Auswertung der Bildpunkte erfolgt vorzugsweise von oben (über der Untersuchungsfläche). Die Verarbeitung zu einem zweidimensionalen Bild erfolgt vorzugsweise mit Hilfe von Computertechniken.

Bei bekannten ELISA-Spot-Verfahren ist man bestrebt, die Farbreaktionen möglichst intensiv und konzentriert auf einen Spot zu erhalten, damit die Zahl der aktiven Zellen möglichst gut erfaßt werden kann.

Bevorzugt werden demgegenüber bei dem angewendeten Verfahren die an der Farbreaktion beteiligten Komponenten in ihrer Menge und/oder Lage gegenüber üblichen Bedingungen reduziert, insbesondere in ihren Konzentrationen, um auch das Farbmaximum einer Zellreaktion in einem für die Intensitätsmessung quantifizierbaren Bereich zu halten.

Erfindungsgemäß werden Maßnahmen ergriffen, wodurch die durch die ausgeschiedenen Stoffe erzeugte Färbung in der Intensität pro Einzelpunkt im Vergleich zum Stand der Technik verringert und/oder in der Flächenausdehnung, d.h. Zahl der Einzelpunkte pro Zelle vergrößert wird. Durch die Vergrößerung der Flächenausdehnung wird insbesondere die Färbung pro Flächeneinheit schwächer und somit Färbungsunterschiede zwischen den einzelnen Flächen und/oder innerhalb einer größeren Fläche deutlicher. Eine Überlappung der Farbflächen der von den Zellen ausgeschiedenen Stoffe stört nicht. Auf diese Weise werden die Farbintensitäten der Einzelpunkte in den technisch meßbaren Bereich geführt. Somit können intensiv gefärbte Flächen, deren Intensität den technisch erfaßbaren Maximalwert überschritten haben, mittels des erfindungsgemäßen Verfahrens aufgrund der größeren Flächenausdehnung einer differenzierenden, insbesondere die Quantität und Qualität der zellulären Reaktion anhand der Menge an ausgeschiedenen Stoffen berücksichtigenden, Messung zugeführt werden. Eine anschließend durchgeführte Messung der Farbintensität über die gefärbte Fläche führt somit zu genauen und damit reproduzierbaren Ergebnissen hinsichtlich der Gesamtaktivität der Zellen. Durch das Verfahren ist es daher auch insbesondere möglich, die Gesamtaktivität von Zellverbänden zu messen, die auf der Untersuchungsfläche zu überlappenden Farbflächen führen können. Auf eine Zählung der aktiven Zellen kommt es dabei nicht mehr an.

Erfindungsgemäß ist es besonders bevorzugt, dass für eine Bindung der Abfangmoleküle an den Träger ein Trägermaterial verwendet wird, das eine geringe Affinität zu den Abfangmolekülen besitzt. Bei der Affinität handelt es sich bevorzugt um Bindungsaffinität, die insbesondere durch kovalente Bindungen, elektrostatische Wechselwirkungen, Wasserstoffbrückenbindungen und/oder hydrophobe Wechselwirkungen, insbesondere Van-der-Waals Kräfte, hergestellt werden kann. Die geringe Affinität des Trägermaterials zu den Abfangmolekülen bewirkt eine geringe Anzahl von immobilisierten Abfangmolekülen auf der Untersuchungsfläche, wodurch die von den Zellen ausgeschiedenen Stoffe einen längeren Diffusionsweg auf der Untersuchungsfläche zurücklegen können, bevor sie mit den Abfangmolekülen reagieren und durch eine geeignete Farbreaktion sichtbar gemacht werden. Die verlängerten Diffusionswege der ausgeschiedenen Stoffe bewirken die bereits oben beschriebene schwächere Färbung pro Flächeneinheit und führen damit zu exakteren und zuverlässigeren Messergebnissen.

Als geeignetes Trägermaterial kann insbesondere Polystyrol, Polyvinylendifluorid (PVDF), Nitrocellulose oder Nylon verwendet werden, wobei Polystyrol aufgrund seiner geringen Bindungsaffinität zu den Abfangmolekülenbesonders bevorzugt ist. Im Falle des Polystyrols kann es sich insbesondere um gespritztes oder gegossenes Polystyrol handeln. Geeignet sind besonders 96-Lochplatten aus Polystyrol.

In einer weiteren Ausführungsform des erfindungsgemäßen Verfahrens kann als weitere Maßnahme zur Verringerung der Intensität pro Einzelpunkt und zur Vergrößerung der Flächenausdehnung mit einer verringerten Konzentration an Abfangmolekülen gearbeitet werden. So wird beim ELlspot-Verfahren insbesondere mit einer Konzentration von Abfangmolekülen von ca. 5 µg/ml gearbeitet. Bei Verwendung kleinerer Konzentrationen wird eine kleinere Menge von Abfangmolekülen auf der Untersuchungsfläche immobilisiert, wodurch größere Spots mit deutlichen Färbungsunterschieden auf der Untersuchungsfläche entstehen. Mit Vorteil erfolgt die Inkubationszeit nach Belegung des Trägers mit den Abfangmolekülen 4 bis 18 h. Nach erfolgter Inkubation werden überschüssige nicht immobilisierte Abfangmoleküle vom Träger vorzugsweise weggespült.

Erfindungsgemäß ist es insbesondere bevorzugt, daß bei der Belegung des Trägers mit einer Konzentration an Abfangmolekülen von 2 bis 3 µg/ml, insbesondere von ca. 2,5 µg/ml, pro 20 mm² gearbeitet wird. Vorzugsweise werden auf der Untersuchungsfläche des Trägers, ca. 1 µg Abfangmoleküle immobilisiert.

In einer weiteren besonders bevorzugten Ausführungsform des erfindungsgemäßen Verfahrens kann die Verringerung der Intensität der Einzelpunkte der durch die ausgeschiedenen Stoffen bedingte Färbung sowie die Vergrößerung der Flächenausdehnung sowohl durch eine Verringerung der Konzentration der Abfangmoleküle als auch durch Verwendung eines Trägermaterials mit geringer Affinität zu den Abfangmolekülen, insbesondere Polystyrol, erzeugt werden.

In manchen Fällen kann es wünschenswert sein, gegebenenfalls zusätzlich zur Messung der Gesamtfarbintensität, die Anzahl von aktiven Zellen auf der Untersuchungsfläche zu messen, wobei sich die Schwierigkeit ergeben kann, daß die in einem Zellverband vorliegenden Zellen aufgrund ihrer funktionalen und/oder strukturellen Verknüpfung und durch die dadurch bedingte gegenseitige Färbungsüberlappung nicht einzeln erfaßbar sind. Dabei kann es vorgesehen sein, daß die Anzahl von Zellen, insbesondere in einem Zellverband, mit Ausscheidung von detektierbaren Stoffen bei sich gegenseitig überlappenden Färbungen dadurch bestimmt wird, daß jeweils nur die den einzelnen Zellen zuzuordnenden Farbmaxima (maximale Farbintensitätswerte) bestimmt werden und diese ausgezählt werden.

Vorzugsweise werden bei dem Verfahren eukaryotische Zellen, insbesondere menschliche und/oder tierische Zellen verwendet, wobei die Zellen vorzugsweise vom lebenden Organismus entnommen werden. Bei den Zellen handelt es sich insbesondere um Blutzellen, beispielsweise immunkompetente Zellen, vorzugsweise Lymphozyten, T-Zellen, insbesondere T-Helferzellen (T_{H}-Zellen), und/oder dendritische Zellen, insbesondere Antigen präsentierende Zellen, beispielsweise Makrophagen. Vorzugsweise werden bei dem Verfahren T-Helferzellen, insbesondere T_{H1}- und T_{H2}-Zellen, verwendet.

Es ist weiterhin vorgesehen, daß die Zellen vor der Messung, insbesondere vor der Fixierung, auf der Untersuchungsfläche des Trägers, angereichert werden. Die Anreicherung der Zellen kann beispielsweise mit Hilfe der Zentrifugation, insbesondere in einem Zuckergradienten, erfolgen. Auf diese Weise ist es insbesondere möglich, die schweren Erythrozyten von den leichteren immunkompetenten Zellen abzutrennen.

Es ist insbesondere vorgesehen, daß die Zellen vor dem Aufbringen auf die Untersuchungsfläche des Trägers gewaschen werden, insbesondere in einer Pufferlösung. Die Zellen werden insbesondere ausgezählt. Bevorzugt werden die Zellen in Form einer Einfachschicht (Monolayer) auf der Untersuchungsfläche des Trägers abgelegt.

Vorteilhafterweise werden als Abfangmoleküle gegen die von den Zellen ausgeschiedenen Stoffe gerichtete Moleküle verwendet. Bevorzugt handelt es sich bei den Abfangmolekülen um Proteine, vorzugsweise um Antikörper. Die Abfangmoleküle können insbesondere Markierungen aufweisen, die einen kolorimetrischen Nachweis erlauben. Vorzugsweise handelt es sich bei den Abfangmolekülen, insbesondere Antikörper, um sogenannte Konjugate, die ein für den kolorimetrischen Nachweis geeignetes Protein, insbesondere ein Enzym, vorzugsweise alkalische Phosphatase oder Peroxidase, beispielsweise Meerrettich-Peroxidase, aufweisen.

In einer weitergehenden Ausführungsform des Verfahrens können als Abfangmoleküle Konjugate mit Gold oder Silber, vorzugsweise Silber, verwendet. Die Gold- oder Silbermarkierungen der Abfangmoleküle können im Rahmen von dem Fachmann geläufigen Nachweisverfahren, beispielsweise ImmunoGold-Verfahren, dazu verwendet werden, auf der Untersuchungsfläche des Trägers gefärbte Flächen zu erzeugen. Vorzugsweise werden Antikörper-Konjugate mit Gold oder Silber, vorzugsweise Silber, verwendet. Als Färbung werden Strahlungen verstanden, die sich von denen des Untergrundes unterscheiden. Hierzu gehören auch Schwarz, Weiß oder Graufärbungen sowie nicht sichtbare Strahlungen.

In einer bevorzugten Ausführungsform werden zur Sichtbarmachung der von den Zellen ausgeschiedenen Stoffe neben den Abfangmolekülen Detektionsmoleküle verwendet. Bei den Detektionsmolekülen handelt es sich vorzugsweise um Antikörper. Die Detektionsmoleküle sind zweckmäßigerweise mit Verbindungen konjugiert, welche einen kolorimetrischen Nachweis erlauben. Bei den Verbindungen handelt es sich insbesondere um Protein, vorzugsweise um Enzyme. So können die Detektionsmoleküle beispielsweise mit Phosphatase oder Peroxydase, insbesondere mit Meerrettich-Peroxydase, konjugiert sein. Weiterhin können die Detektionsmoleküle mit Gold oder Silber konjugiert sein. Bezüglich weiterer Einzelheiten und Merkmale wird auf die bisherige Beschreibung verwiesen.

In einer weiteren Ausführungsform des Verfahrens wird ein Farbreagenz auf die auf der Untersuchungsfläche des Trägers fixierten Zellen gegeben. Vorzugsweise handelt es sich bei dem Farbreagenz um ein Substrat eines mit dem Abfangmolekül konjugierten Enzyms, insbesondere Para-Nitrophenylphosphat, Carbazol oder BromChlor-Indolyl-Phosphat. Derartige Ausführungsformen zur Erzeugung einer Farbreaktion sind dem Fachmann bekannt, so daß auf eine weitergehende Beschreibung an dieser Stelle verzichtet wird.

In einer weitergehenden Ausbildung des Verfahrens handelt es sich bei den ausgeschiedenen Stoffen insbesondere um Botenstoffe, vorzugsweise Zytokine. Erfindungsgemäß ist es besonders bevorzugt, daß es sich bei den ausgeschiedenen Stoffen um mindestens einen Vertreter aus der Gruppe, umfassend Interleukin 2, Interleukin 4, Interleukin 5, Interleukin 10, Gamma-Interferon und/oder TNFβ (Tumor Necrosis Factor β) handelt, wobei Gamma-Interferon gemäß der vorlegenden Erfindung besonders bevorzugt ist.

Die Ausscheidung der Stoffe wird insbesondere durch immunologische Fremdstoffe, sogenannte Antigene, auf der Untersuchungsfläche veranlasst. Bei den Antigenen kann es sich insbesondere um vollständige Erreger, beispielsweise Viren und/oder Bakterien handeln. In einer besonderen Ausführungsform des Verfahrens handelt es sich bei den Antigenen um sogenannte Epitope (Fragmente), insbesondere um Peptide, beispielsweise mit einer Aminosäurenlänge von 9 bis 11 Aminosäuren. Zusätzlich können durch die Anwesenheit von dendritischen Zellen, insbesondere Makrophagen, auf der Untersuchungsfläche Epitope durch die Phagozytose von vollständigen Erregern erzeugt werden, die vorzugsweise auf der Oberfläche von sogenannten antigen-präsentierenden Zellen präsentiert werden.

Die Untersuchungsfläche des Trägers ist vorzugsweise eben. Dies ist besonders bevorzugt, da auf diese Weise lokale Konzentrationsänderungen auf der Untersuchungsplatte vermieden werden können. Weiterhin kann es sich bei der Untersuchungsfläche insbesondere um eine runde Fläche, beispielsweise um den Lochboden einer Lochplatte handeln. Bei den Lochplatten handelt es sich um kommerziell erhältliche Mikrotiterplatten, die eine unterschiedliche Anzahl von Löchern (Vertiefungen bzw. Wells) aufweisen können. Bevorzugt wird im Rahmen des erfindungsgemäßen Verfahrens eine Lochplatte mit 96 Löchern (96-Lochplatte) verwendet.

Die 96-Lochplatten haben bevorzugt einen Lochdurchmesser am Boden von ca. 5 mm, was einer Grundfläche von etwa 20 mm² entspricht. In einigen Fällen kann es erforderlich sein, mit kleineren Untersuchungsflächen zu arbeiten. Erfindungsgemäß kann es daher vorgesehen sein, Grundflächen zu verwenden, die kleiner als 15 mm², insbesondere kleiner als 10 mm², sind. Die bevorzugte Fläche von 20 mm² dient insbesondere dazu, den Zugang der für die üblichen Lochplatten, insbesondere 96-Lochplatten, entwickelten Einrichtungen, insbesondere zum automatischen Füllen, Spülen, Entnehmen und Messen, verwenden zu können.

Bei den Erkrankungen, die mit Hilfe des Verfahrens diagnostiziert und/oder deren Verlauf beobachtet werden können, handelt es ich insbesondere um Infektionserkrankungen, Autoimmunerkrankungen und/oder Allergien. Vorzugsweise handelt es sich bei den Infektionserkrankungen um Tuberkulose, Zytomegalievirus (CMV), Borreliose, Eppstein-Barr-Virus (EBV), Herpes, Influenza und/oder Hepatitis C. Bei den Autoimmunerkrankungen handelt es sich vorzugsweise um rheumatoide Arthritis.

Das erfindungsgemäße Verfahren erlaubt durch die Beeinflussung, insbesondere Verringerung, der Intensitäten der gefärbten Flächen sowie durch Vergrößerung der Flächenausdehnung der gefärbten Flächen auf der Untersuchungsfläche eine exakte und zuverlässige Beurteilung insbesondere der quantitativen Gesamtaktivität von Zellen. Durch die verbesserte quantitative Aussagekraft der Messergebnisse kann eine verbesserte Einschätzung des sogenannten Immunstatus eines Patienten vorgenommen werden. So kann mit Hilfe des Verfahrens insbesondere beurteilt werden, ob die Betroffenen eine akute Infektion oder eine bereits abklingende Infektion aufweisen oder sogar an einer chronischen Erkrankung leiden. Mit Hilfe des Verfahrens ist es außerdem möglich, die Gesamtaktivität von Zellverbänden zu bestimmen. Ein weiterer Vorteil ergibt sich aus der Tatsache, daß das erfindungsgemäße Verfahren mit verringerten Konzentrationen von Abfangmolekülen, insbesondere teuren Antikörpern, durchgeführt werden kann.

### Beispiel

Auf den Lochboden (Durchmesser: 20 mm²) werden ca. 2,5 µg eines Antikörpers, der mit alkalischer Phosphatase konjugiert ist, hinzugegeben. Die Inkubation erfolgt bei 37 °C während 4 bis 12 Stunden, vorzugsweise über Nacht. Anschließend werden nicht gebundene Antikörper von dem Lochplattenboden einer 96-Lochplatte abgespült, wobei sich ca. 1 µg Antikörper danach an jedem Lochboden befinden. Anschließend werden ca. 50 µl einer aufgereinigten Zellfraktion (mit Hilfe von Zentrifugation) auf den Lochboden aufgebracht. Dies ergibt eine Monolayer-Schicht aus ca. 200.000 Zellen pro Loch. Die Inkubation mit einer 50 µl Antigensuspension erfolgt ebenfalls bei 37 °C während 18 Stunden, vorzugsweise über Nacht. Nach Zugabe von p-Nitrophenlyphosphat wird die Aktivität aller Zellen auf der Untersuchungsfläche anhand der auftretenden Gesamtfärbung ermittelt.

### Diagnose einer latenten oder akuten Tuberkulose:

Für die Ermittlung des Immunstatus bei Vorliegen einer Tuberkulose werden insgesamt 8 Ansätze getätigt, wobei der 1. Ansatz dem Leerwert entspricht (keine Aktivität: ca. 0 bis 200 Einheiten). Der 2. Ansatz enthält das Pokeweed Antigen, welches ca. 0,5 bis 2 % aller auf der Untersuchungsfläche vorhandenen T-Zellen stimuliert. Eine gemessene Aktivität von ca. 100.000 entspricht der bei einem Patienten zu beobachtenden Aktivität, wobei der Patient über ein normal gut funktionierendes Immunsystem verfügt. Wird eine Aktivität von weniger als 60.000 gemessen, so leidet der Patient an einer Immunschwäche. Diese Aussage ist besonders von Bedeutung, da viele Tuberkulose-Patienten infolge der Therapie an einer Immunsupression leiden. Der 3. Ansatz enthält das Mykobakterium Antigen. Eine gemessene Aktivität von bis zu 500 deutet darauf hin, daß der Patient Tuberkulose negativ ist. Im übrigen ist kein früherer Impfschutz damit nachweisbar. Eine gemessene Aktivität von mehr als 1.000 zeigt eine Tuberkulose bei dem Betroffenen an. Der 4. Ansatz enthält die Tuberkulose Antigene ESAT6 und CFP10. Eine negative Aktivität kann entweder keine akute oder eine latente Tuberkulose bedeuten, wobei eine Aktivität bis ca. 800 (geringe Aktivität) auf eine latente Tuberkulose schließen lässt. Alternativ kann das Ergebnis aber auch als eine bereits abgeklungene Tuberkulose beurteilt werden. Somit ist es möglich, latente Tuberkuloseformen zu diagnostizieren. Eine Aktivität über 1.000 weist dagegen eine klare Tuberkulose, vermutlich eine akute Infektion, nach. Bei den Ansätzen 5, 6 und 7 handelt es sich jeweils um den gleichen Ansatz wie in Ansatz 4 (sogenannte Wiederholungsansätze). Der Ansatz 8 dient als Leerwert oder weitere Kontrolle.

### Immunstatus zur Erfolgskontrolle bei Impfungen

Zur Ermittlung des Immunstatus für eine Erfolgskontrolle bei einer Impfung werden ebenfalls 8 Ansätze getätigt. Ansatz 1 entspricht dem Leerwert (keine Aktivität: ca. 0 bis 200 Units, entspricht gutem Leerwert). Der Ansatz 2 enthält das Pokeweed Antigen. Der Ansatz 3 weist einen Mischung aus Zytomegalievirus, Eppstein-Barr-Virus und Flue Influenza Antigenen auf (CEF), die bei vielen Patienten eine spezifische T_{H-1}-induzierte Immunantwort hervorruft, da die meisten Personen mit mindestens einem der oben angeführten Erregern schon einmal infiziert waren. Eine gemessene Aktivität von bis zu 500 besagt, daß der Patient in Bezug auf die CEF enthaltenen Erregern negativ ist und auch an keiner frühere Infektion gelitten hat. Eine gemessene Aktivität von mehr als 1.000 zeigt, daß das Immunsystem des Betroffenen auf mindestens einen der in CEV enthaltenen Erregern spezifisch reagiert hat. Der 4. Ansatz enthält die Komponenten des Impfstoffes, wobei eine negative Aktivität einen misslungenen Impferfolg nachweist. Eine gemessene Aktivität bis 800 (geringe Aktivität) spricht für einen lediglich geringen Impferfolg, der vermutlich zu keinem wirksamen Impfschutz führt. Eine gemessene Aktivität über 1.000 indiziert einen Impfschutz. Bei den Ansätzen 5, 6 und 7 handelt es sich um sogenannte Wiederholungsansätze. Der Ansatz 8 kann als Leerwert oder weitere Kontrolle dienen.

### Immunstatus zur Abschätzung der Funktionalität eines normalen Immunsystems

Zur Ermittlung des Immunstatus bei der Messung der normalen Funktionalität des Immunsystems werden insgesamt 8 Ansätze getätigt. Der 1. Ansatz dient als Leerwert. Der Ansatz 2 enthält das Pokeweed Antigen. Der Ansatz 3 kann einen virulenten Peptidpool, beispielsweise von Eppstein-Barr-Virus (EBV) und Zytomegalievirus (CMV), enthalten, wobei eine gemessene Aktivität von mehr als 1.000 eine gute Aktivierbarkeit nachweist und eine gemessene Aktivität von weniger als 1.000 eine schwache Immunreaktion gegen die oben genannten Viren belegt. Der Ansatz 4 enthält die Komplettantigene EBV und CMV (ganze Viren), wobei eine gemessene Aktivität von ca. 800 eine gering positive Reaktion auf die genannten Viren nachweist. Eine gemessene Aktivität von mehr als 1.000 spricht für eine klare Reaktion auf EBV und CMV. Wird in Ansatz 3 eine hohe Aktivität gemessen, in Ansatz 4 aber eine lediglich schwache Aktivität oder sogar eine negative Aktivität, so kann auf einen Immundefekt, der die Prozessierfähigkeit (Phagozytose von Komplettantigenen, Verdauung des Komplettantigens zu Epitopen sowie deren Präsentation an der Oberfläche von sogenannten Antigen-präsentierenden Zellen) betrifft, geschlossen werden. Das bedeutet, daß insbesondere die Funktion der dendritischen Zellen gestört ist. Ansatz 5 enthält bakterielle Antigene, beispielsweise PPD (Purified Protein Derivative, Tuberkulin) oder Tetanus Toxoid. Der Ansatz kann als Kontrolle für die Reaktivität gegen Bakterien dienen, wobei eine analoge Beurteilung wie bei Ansatz 4 anzuwenden ist. Ansatz 6 enthält Pilzantigene, beispielsweise von Candida oder Aspergillus, und dient als Kontrolle des Immunsystems auf Reaktivität gegen Pilze oder Hefen. Ansatz 7 enthält beispielsweise ein Allergen. Ansatz 8 dient zur Messung der NK (Natural Killer, Zellen) Zellaktivität, wobei zur Stimulierung sogenannte Sphingolipide (stimulieren nur NK Zellen) oder NK aktivierende Antikörper benutzt werden. Mit Hilfe von Ansatz 8 können immunologische Reaktionen gegen entartete Zellen beurteilt werden.

### Immunstatus zur Prognose oder Kontrolle von chronischen Erkrankungen am Beispiel von rheumatoider Arthritis

Es werden insgesamt 8 Ansätze getätigt, um den Immunstatus zur Messung der Funktionalität zu ermitteln. Ansatz 1 entspricht dem Leerwert. Ansatz 2 enthält das Pokeweed Antigen. Der Ansatz 3 weist einen von dem Eppstein-Barr-Virus stammenden Peptidpool auf und dient zum Nachweis einer Immunreaktion gegen Viren und Autoantigene, wobei eine gemessene Aktivität über 1.000 eine gute Aktivierbarkeit und möglicherweise eine autoimmunologische Erkrankung nachweist. Ansatz 4 enthält EBV Peptide, die Autoantigene darstellen, wobei eine gemessene Aktivität von über 1.000 eine eindeutige autoimmunologische Reaktion nachweist, während eine Aktivität unterhalb von 800 auf eine lediglich gering positive autoimmunologische Reaktion hindeutet. Die Ansätze 5 und 6 entsprechen den Ansätzen 3 und 4, können gegebenenfalls aber auch mit anderen Antigenen inkubiert sein. Im Falle des Ansatzes 7 wird ein spezielles Antigen, welches für den Betroffenen individuell ist, beispielsweise ein Heatshock-Protein, eingesetzt. Ansatz 8 dient der Messung des speziellen Antigens CCP (Cyclisches Citrulliniertes Peptid).

### Immunstatus zur Abschätzung einer chronischen Erkrankung versus vollständiger Eliminierung des Erregers

Es werden insgesamt 8 Ansätze für die Ermittlung des Immunstatus getätigt, wobei Ansatz 1 als Leerwert dient. Ansatz 2 weist das Pokeweed Antigen auf. Ansatz 3 weist spezielle Antigene, beispielsweise Hepatitis C-Antigene, auf. Eine gemessene Aktivität von über 1.000 weist eine eindeutige Reaktion auf die Antigene nach, während eine gemessene Aktivität unterhalb von 800 lediglich eine gering positive Reaktion nachweist. Ansatz 4 entspricht Ansatz 3, wobei als ausgeschiedene Stoffe Interleukin 4 und Interleukin 5 gemessen werden und eine positive Reaktion ab einer gemessenen Aktivität von ca. 300 nachgewiesen wird. Ansatz 5 entspricht Ansatz 3, wobei lediglich Interleukin 10 als ausgeschiedener Stoff gemessen wird und von einer positiven Reaktion ab einer gemessenen Aktivität von 2.000 gesprochen werden kann. Die Ansätze 6, 7 und 8 entsprechen den Ansätzen 3, 4 und 5. Bei den Interleukinen 4, 5 und 10 handelt es sich um sogenannte suprimierende Zytokine. Sind diese Zytokine im Vergleich zu Gamma-Interferon vermehrt ausgeschüttet, so liegt das Immunsystem insgesamt suprimiert vor und das Risiko einer chronischen Infektion steigt.

## Patentansprüche

1. Verfahren zur Bestimmung der Menge von zellulär ausgeschiedenen detektierbaren Stoffen, insbesondere zur Diagnose und/oder Verfolgung der Therapie von Erkrankungen, bei welchem auf mindestens einer Untersuchungsfläche eines Trägers von Zellen ausgeschiedene Stoffe mittels Abfangmolekülen anhand von gefärbten Flächen sichtbar gemacht werden, indem die Flächen in Einzelpunkte zerlegt und diese verarbeitet werden, **dadurch gekennzeichnet, daß** die Intensität der gesamten Färbung auf der Untersuchungsfläche quantitativ gemessen wird, wobei die Untersuchungsfläche in eine Vielzahl von Einzelpunkten zerlegt wird, die Farbintensität jedes Einzelpunktes getrennt gemessen wird und die gemessenen Intensitätswerte addiert werden und die durch die ausgeschiedenen Stoffe erzeugte Färbung in der Intensität pro Einzelpunkt verringert und/oder in der Flächenausdehnung vergrößert wird.

2. Verfahren nach Anspruch 1, **dadurch gekennzeichnet, daß** für eine Bindung der Abfangmoleküle an den Träger ein Trägermaterial verwendet wird, das eine geringe Affinität zu den Abfangmolekülen besitzt, wobei als Trägermaterial Polystyrol, Polyvinylendifluorid (PVDF), Nitrocellulose oder Nylon verwendet wird, vorzugsweise Polystyrol.

3. Verfahren nach Anspruch 1 oder 2, **dadurch gekennzeichnet, daß** bei der Belegung des Trägers mit einer Konzentration an Abfangmolekülen von 2 bis 3 µg/ml, insbesondere ca. 2,5 µg/ml, gearbeitet wird.

4. Verfahren nach einem der vorhergehenden Ansprüche, **dadurch gekennzeichnet, daß** auf der Untersuchungsfläche, insbesondere auf dem Lochboden einer 96-Lochplatte, ca. 1 µg Abfangmoleküle gebunden werden.

5. Verfahren nach einem der vorhergehenden Ansprüche, **dadurch gekennzeichnet, dass** zur Sichtbarmachung der von den Zellen ausgeschiedenen Stoffe neben den Abfangmolekülen Detektionsmoleküle verwendet werden.

6. Verfahren nach einem der vorhergehenden Ansprüche, **dadurch gekennzeichnet, daß** es sich bei den von den Zellen ausgeschiedenen Stoffen um mindestens einen Vertreter aus der Gruppe, umfassend Interleukin 2, Interleukin 4, Interleukin 5, Interleukin 10, γ-Interferon und/oder TNFβ (Tumour Necrosis Factor beta), handelt, vorzugsweise um γ-Interferon.

7. Verfahren nach einem der vorhergehenden Ansprüche, **dadurch gekennzeichnet, daß** es sich bei den Erkrankungen um Tuberkulose, CMV (Zytomegalie-Virus), Borreliose, EBV (Epstein-Barr-Virus), Herpes, Influenza und/oder Hepatitis C handelt.

## Claims

1. Method of determining the amount of detectable substances excreted by cells, in particular for the diagnosis of and/or following the therapy of disorders, in which, on at least one examination surface of a carrier, substances excreted by cells are visualized by means of capture molecules on the basis of stained areas, by disrupting the areas into single points and processing these, **characterized in that** the intensity of the entire colouring on the examination surface is measured quantitatively, wherein the examination surface is disrupted into a multiplicity of single points, the colour intensity of each single point is measured separately and the measured intensity values are added, and the colouring generated by the excreted substances is decreased in intensity per single point and/or increased in area extent.

2. Method according to Claim 1, **characterized in that**, for binding of the capture molecules to the carrier, a carrier material is used which has a low affinity to the capture molecules, wherein the carrier material used is polystyrene, polyvinylene difluoride (PVDF), nitrocellulose or nylon, preferably polystyrene.

3. Method according to Claim 1 or 2, **characterized in that**, when the carrier is coated, a concentration of capture molecules of 2 to 3 µg/ml, in particular approximately 2.5 µg/ml, is employed.

4. Method according to one of the preceding claims, **characterized in that** approximately 1 µg of capture molecules are bound on the examination surface, in particular on the well plate of a 96-well plate.

5. Method according to one of the preceding claims, **characterized in that**, for visualization of the substances excreted by the cells, in addition to the capture molecules, detection molecules are used.

6. Method according to one of the preceding claims, **characterized in that** the substances excreted by the cells are at least one member of the group consisting of interleukin 2, interleukin 4, interleukin 5, interleukin 10, γ-interferon and/or TNFβ (tumour necrosis factor beta), preferably γ-interferon.

7. Method according to one of the preceding claims, **characterized in that** the disorders are tuberculosis, CMV (cytomegalovirus), borreliosis, EBV (Epstein-Barr virus), herpes, influenza and/or hepatitis C.

## Revendications

1. Procédé pour déterminer la quantité de substances détectables excrétées par les cellules, en particulier pour le diagnostic et/ou le suivi du traitement de maladies, dans lequel, sur au moins une surface d'essai d'un support, des substances excrétées par des cellules sont visualisées grâce à des molécules de capture, à l'aide de surfaces colorées, les surfaces étant décomposées en points individuels, et ces derniers étant transformés, **caractérisé en ce qu'**on mesure quantitativement l'intensité de la coloration totale sur la surface d'essai, la surface d'essai étant décomposée en un grand nombre de points individuels, l'intensité de couleur de chaque point individuel étant mesurée séparément, et les valeurs mesurées de l'intensité étant ajoutées, et la coloration produite par les substances excrétées subit une diminution de son intensité par points individuels et/ou un agrandissement de son étendue en surface.

2. Procédé selon la revendication 1, **caractérisé en ce que**, pour une liaison des molécules de capture au support, on utilise un matériau support qui présente une faible affinité pour les molécules de capture, auquel cas on utilise en tant que matériau support du polystyrène, du poly(difluorure de vinylidène) (PVDF), de la nitrocellulose ou du nylon, de préférence du polystyrène.

3. Procédé selon la revendication 1 ou 2, **caractérisé en ce que**, lors de l'occupation du support, on travaille avec une concentration des molécules de capture de 2 à 3 µg/ml, en particulier d'environ 2,5 µg/ml.

4. Procédé selon l'une des revendications précédentes, **caractérisé en ce qu'**on lie environ 1 µg de molécules de capture sur la surface d'essai, en particulier sur le fond des puits d'une plaque à 96 puits.

5. Procédé selon l'une des revendications précédentes, **caractérisé en ce que**, pour visualiser les substances excrétées par les cellules, on utilise des molécules de détection en plus des molécules de capture.

6. Procédé selon l'une des revendications précédentes, **caractérisé en ce que**, pour ce qui concerne les substances excrétées par les cellules, il s'agit d'au moins un représentant du groupe comprenant l'interleukine 2, l'interleukine 4, l'interleukine 5, l'interleukine 10, l'interféron γ et/ou le TNFβ (facteur de nécrose tumorale bêta), de préférence de l'interféron γ.

7. Procédé selon l'une des revendications précédentes, **caractérisé en ce que**, pour ce qui concerne les maladies, il s'agit de la tuberculose, du CMV (cytomégalovirus), de la borréliose, de l'EVB (virus d'Epstein-Barr), de l'herpès, de la grippe et/ou de l'hépatite C.
